# EUROPEAN PATENT SPECIFICATION

(11) **EP 2 827 793 B1**
(45) Date of publication and mention of the grant of the patent: **31.01.2018**
(21) Application number: 13764568.5
(22) Date of filing: 19.03.2013
(51) Int. Cl.: A61B 1/00, G06T 19/00, H04N 13/02

(54) **IMAGE SYSTEM FOR SURGERY AND METHOD FOR IMAGE DISPLAY**
BILDSYSTEM FÜR DIE CHIRURGIE UND VERFAHREN ZUR BILDANZEIGE
SYSTÈME D'IMAGE POUR UNE OPÉRATION CHIRURGICALE ET PROCÉDÉ D'AFFICHAGE D'IMAGES

(30) Priority: 21.03.2012 JP 2012063510
(43) Date of publication of application: 28.01.2015
(73) Proprietor: Olympus Corporation, Tokyo 192-8507 (JP)
(72) Inventor: KONNO, Osamu, Hachioji-shi, Tokyo 192-8507 (JP); KOBAYASHI, Hiroyoshi, Hachioji-shi, Tokyo 192-8507 (JP); IKEDA, Hiromu, Hachioji-shi, Tokyo 192-8507 (JP); KIKUCHI, Satoru, Hachioji-shi, Tokyo 192-8507 (JP); MIYAZAKI, Yasuhiro, Hachioji-shi, Tokyo 192-8507 (JP); NICHOGI, Masao, Hachioji-shi, Tokyo 192-8507 (JP); YOSHIMURA, Katsuhiko, Hachioji-shi, Tokyo 192-8507 (JP)
(74) Representative: Gunzelmann, Rainer
(86) International application number: PCT/JP2013/058890
(87) International publication number: WO 2013/141404

(56) References cited:
- EP-A1- 2 328 354
- JP-A- 2003 066 336
- JP-A- 2004 065 804
- JP-A- 2004 305 367
- JP-A- 2005 066 080
- US-A1- 2008 151 041
- US-A1- 2011 228 049

## Description

### {Technical Field}

The present invention relates to an image system for surgery.

### {Background Art}

Conventionally, stereoscopic endoscope devices are known that display parallax images of an object acquired by a plurality of cameras disposed on a distal end of insertion parts of the device to allow an observer to stereoscopically observe the object while performing treatment introduced via a channel to the front of the distal end of the insertion part by a treatment device (for example, see PTL 1.)

Such a stereoscopic endoscope device allows stereoscopic observation by parallax images in a normal state, and provides a two-dimensional image of a part of range in which a treatment device positioned at a closest distance appears within the parallax images where it is detected that the treatment device is inserted into the channel.

### {Citation List}

{PTL 1} Japanese Patent No. 4170042

### {Technical Problem}

The stereoscopic endoscope device of PTL 1 requires the operator to perform stereoscopic observation by parallax images all the time under the state where the treatment devic is not introduced into a body, and inconveniently causes eyestrain accompanied by the stereoscopic observation where the entire surgery process including the search for an affected area continues for a long time.

JP 2004/065804 concerns a technique to stereo-observe an object in an effective area without making a display of a close range area where a treatment implement is present disturb a three-dimensional view. A video of an object to be observed is three-dimensionally observed by a pair of right and left CCDs, and the treatment implement is guided to a top via a treatment implement insertion channel. It is detected by a photo-interrupter that the treatment implement is inserted to the treatment implement insertion channel. When the insertion of the treatment implement is detected, under control of two image memories by a memory control part, 2D video is formed in the close range area, and 3D video is formed in the other area. Thus, the treatment implement displayed in the close range area is prevented from disturbing the stereo-observation.

The present invention is made in view of the aforementioned circumstances, and an object of the present invention is to provide an image system for surgery and a method for image display with the same that can ease fatigue in a surgery.

### {Solution to Problem}

To achieve the above-described objective, the present invention provides following solutions.

One aspect of the present invention provides an image system for surgery, the image system comprising: a three- dimensional imaging unit that acquires two images having parallax by capturing an image of an operation target portion a display unit that displays two images acquired by the three dimensional imaging unit individually for both left and right eyes, or displays a same image for both left and right eyes, or displays a same image for both left and right eyes; an operating state detection unit that detects that a treatment device or the three-dimensional imaging unit that performs treatment on the operation target portion is in an operating state in which to initiate treatment on the operation target portion; and a display control unit that switches from displaying a same one image on the display unit to displaying two images on the display unit when the operating state detection unit detects that the three-dimensional imaging unit or the treatment device is in the operating state in which to initiate treatment on the operation target portion.

According to the present aspect, the operation target portions are imaged by the three-dimensional imaging unit and two images having parallax are acquired. In a state in which the operator is not operating the treatment device for performing treatment on the operation target portion or the three-dimensional imaging unit, the display control unit controls the display unit so as to display a same image acquired from an image acquired by the three-dimensional imaging unit on both left and right eyes. This allows the operator to observe the operation target portion by a two-dimensional image with less burden on the eyes.

On the other hand, where the operator operates the treatment device or the three-dimensional imaging unit and it is detected by the operating state detection unit that the treatment device or the three-dimensional imaging unit is in an operating state in which to initiate treatment on the operation target portion, the display control unit controls the control unit so as to display two images acquired by the three-dimensional imaging unit individually for both left and right eyes. This allows the operator to appropriately perform treatment on the operation target portion by the treatment device while acquiring information in the depth direction of the operation target portion with a stereoscopic image.

In other words, since, according to the present aspect, a stereoscopic image is displayed only in the situation in which to actually perform treatment on an operation target portion, and in other situation, a two-dimensional image is displayed. Therefore, the operator needs not continuously observe the stereoscopic image for an extended period, and it is possible to ease fatigue in a surgery.

In the above-described aspect, the operating state detection unit may be configured to detect displacement, velocity or acceleration of the treatment device.

With this configuration, it is possible to easily detect whether the treatment device is in an operating state in which to initiate the treatment on the operation target portion. In other words, when the treatment device is displaced, when the treatment device has moved with a velocity of predetermined magnitude or more, or acceleration of a predetermined value or more has worked on the treatment device, this is due to an operation by the operator to initiate the treatment on the operation target portion. In this case, by switching the observation to that with a stereoscopic image, it is possible to perform appropriate treatment by the treatment device on the operation target portion.

Further, in the above-described aspect, the operating state detection unit may determine that the treatment device is in the operating state in which to initiate the treatment on the operation target portion when the operating state detection unit processes any of the images acquired by the three-dimensional imaging unit, and the treatment device is detected within the image.

With this configuration, it is possible, without providing a separate sensor for detecting an operating state of a treatment device, etc., to detect an operating state of a treatment device from an image acquired by the three-dimensional imaging unit.

Further, in a configuration in which displacement, velocity or acceleration of the above-described treatment device is detected, the operating state detection unit may process any of the images acquired by the three-dimensional imaging unit and calculate displacement, velocity or acceleration of the treatment device within the image, and determine that the treatment device is in the operating state in which to initiate the treatment on the operation target portion when the calculated displacement, velocity or acceleration exceeds a predetermined threshold.

With this configuration, it is possible to achieve stereoscopic observation where the treatment device has moved within the image on performing treatment on the operation target portion.

Further, in the above-described aspect, the operating state detection unit may be provided on an operation unit for operating the treatment device, and detect contact on the operation unit or the operation of the operation unit.

With this configuration, since the contact or operation on the operation unit to which the intention of the operator is initially conveyed is detected, it is possible to rapidly perform switching from a two-dimensional image to a stereoscopic image.

Further, in the above-described configuration with the operation unit, the operation unit may be provided on a master device disposed at a position remote from the treatment device and operating the treatment device by remote operation.

With this configuration, the operator can observe operation target portion with a two-dimensional image even when treatment is performed by observation from a remote location, where an affected area to be subjected to treatment is found and the operation unit provided in the master device is operated, contact or operation on the operation unit is detected to switch display to a stereoscopic image. Therefore, it is possible to perform appropriate treatment with the treatment device with an image of the operation target portion having depth.

In the above aspect, the operating state detection unit may detect an amount of displacement or a direction of displacement of the operation unit, and in a case where the detected amount of displacement exceeds a predetermined threshold or where it is detected that the direction of displacement is a predetermined direction of displacement, determine that the treatment device is in an operating state in which to initiate treatment on the operation target portion.

With this configuration, it is possible to more reliably determine whether the operator has initiated treatment on the operation target portion by the amount of displacement or the direction of displacement of the operation unit.

Further, in the above-described aspect, the operating state detection unit may be provided on a trocar for allowing insertion of the treatment device from outside of a body to inside of the body, and when a distal end of the treatment device is inserted inside of the body via the trocar, determine that the treatment device is in an operating state in which to initiate treatment on the operation target portion.

With this configuration, it is possible to more reliably determine that the treatment device is in a preparatory state in which to initiate the treatment on the operation target portion by detecting that the treatment device is inserted inside of the body via the trocar.

Further, in the above-described aspect, the operating state detection unit may measure a distance between a distal end position of the treatment device and the operation target portion, and where the measured distance is smaller than a predetermined threshold, determine that the treatment device is in the operating state in which to initiate treatment on the operation target portion.

With this configuration, when the operator is about to perform treatment on the operation target portion, the treatment device is placed closed to the operation target portion. Therefore, it is possible to measure a distance between the operation target portion and the distal end of the treatment device and where the distance becomes smaller than a predetermined threshold, it can be more reliably detected that the treatment device is in an operating state in which to initiate treatment on the operation target portion.

In the above-described aspect, the three-dimensional imaging unit may include a zooming optical system, the operating state detection unit may detect a magnification of the zooming optical system, and determine that the treatment device is in an operating state in which to initiate treatment on the operation target portion when it is detected that the magnification exceeds a predetermined threshold.

With this configuration, since, when the operator is to perform treatment on the operation target portion, the operator increases the magnification of zooming optical system to perform more detailed observation of the operation target portion, it is possible that the three-dimensional imaging unit can detect more reliably that the treatment device is in an operating state in which to initiate treatment on the operation target portion when the magnification of the zooming optical system has become greater than a predetermined threshold.

In the above-described aspect, a storage unit that stores dominant eye information on which eye of the operator the dominant eye is, and the display control unit, where it is not detected that the three-dimensional imaging unit or the treatment device is in the operating state in which to initiate treatment on the operation target portion, displays the image on the dominant eye side on the display unit based on the dominant eye information stored in the storage unit.

With this configuration, it is possible to prevent the range of operation target portion which is the focus of the attention from being offset, and it is possible for the operator to perform the treatment without having an uncomfortable feel where displaying is switched from a two- dimensional image to a stereoscopic image.

Another aspect not forming part of the present invention provides a method for image display, comprising: detecting whether a treatment device that performs treatment on an operation target portion or an imaging unit that acquires two images having parallax by capturing an image of the operation target portion is in an operating state in which to initiate treatment on the operation target portion, and when it is detected that the
treatment device or the imaging unit is not in the operating state in which to initiate treatment on the operation target portion, displaying only one of images acquired by the imaging unit for both left and right eyes, and when it is detected that the treatment device or the imaging unit is in an operating state in which to initiate the treatment on the operation target portion, displaying two images acquired by the imaging unit individually for both left and right eyes.

### {Advantageous Effects of Invention}

According to the present invention, an effect is achieved that can ease fatigue in a surgery.

### {Brief Description of Drawings}

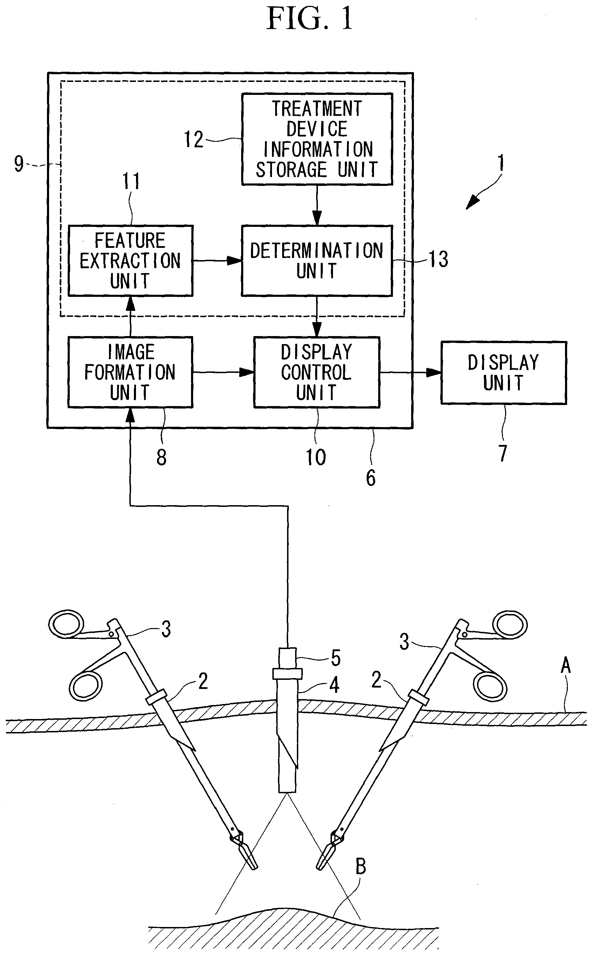

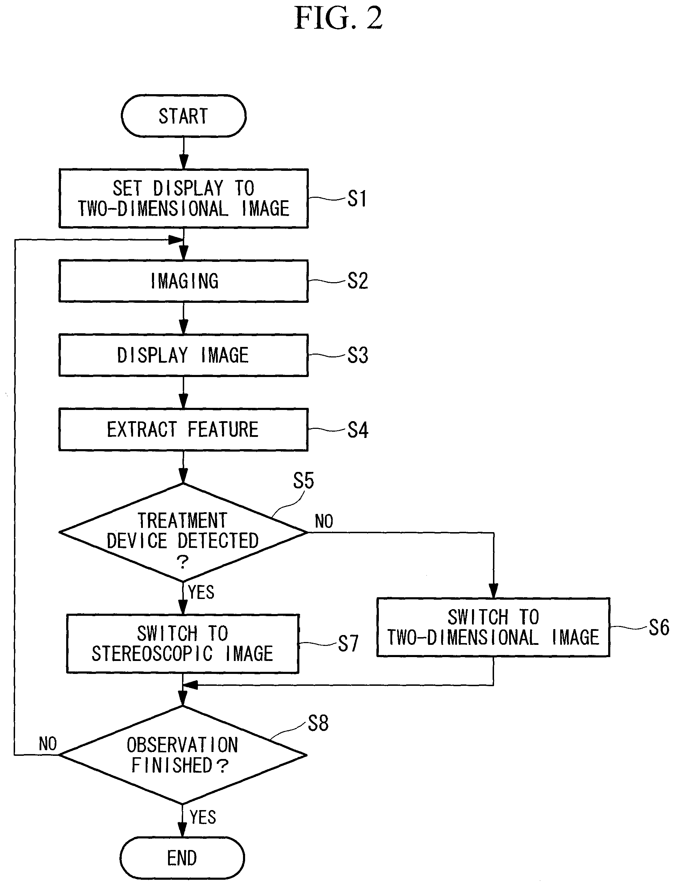

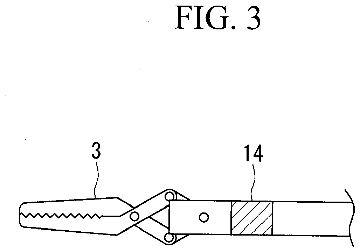

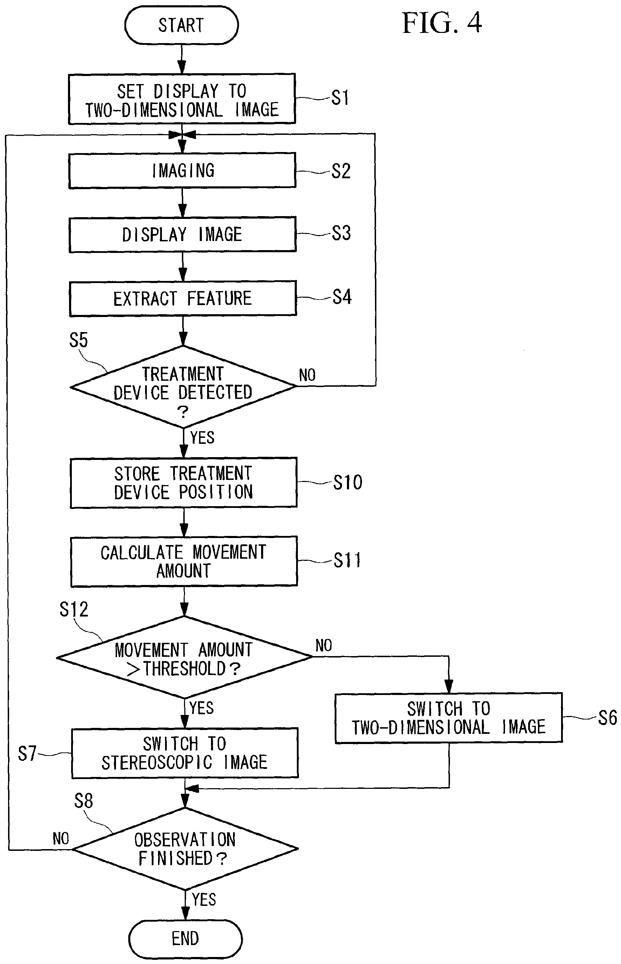

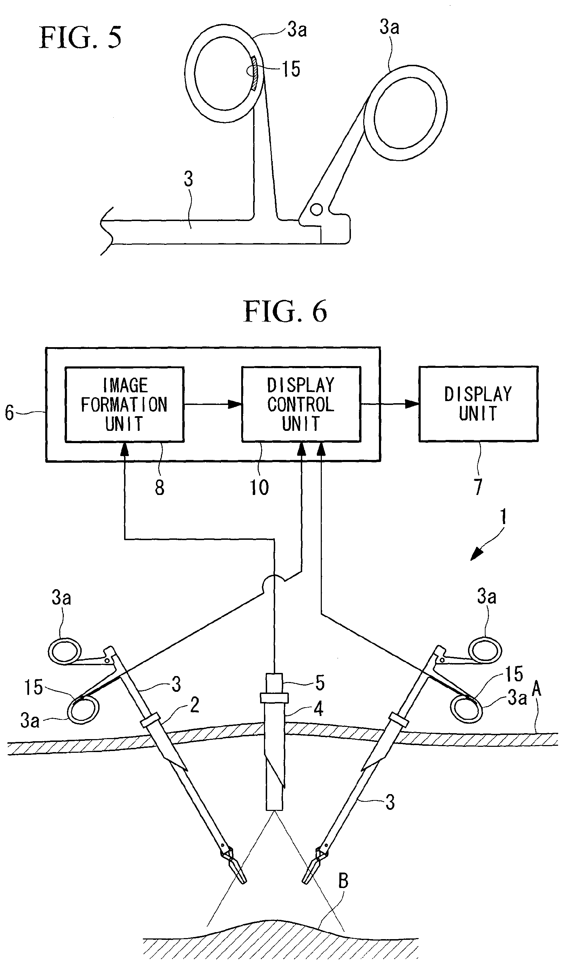

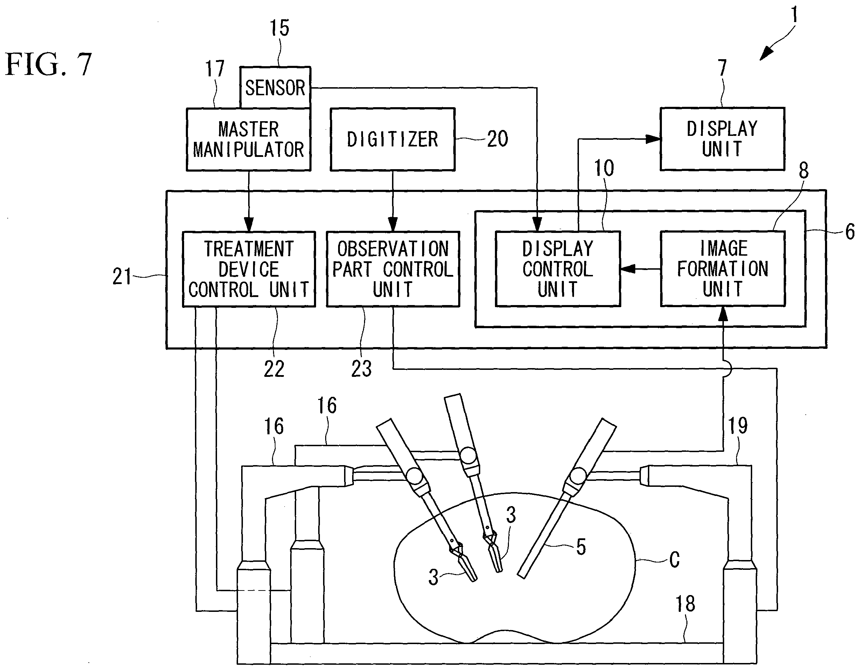

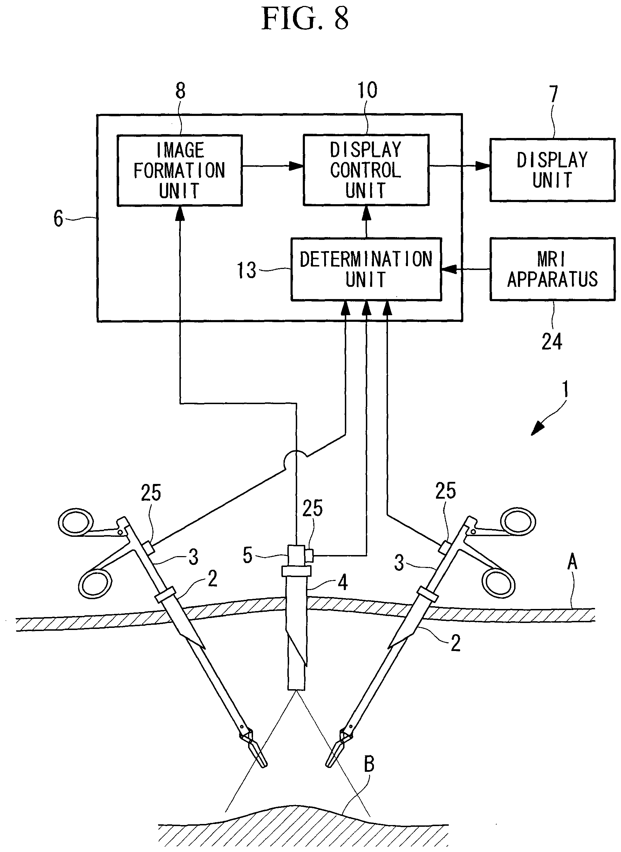

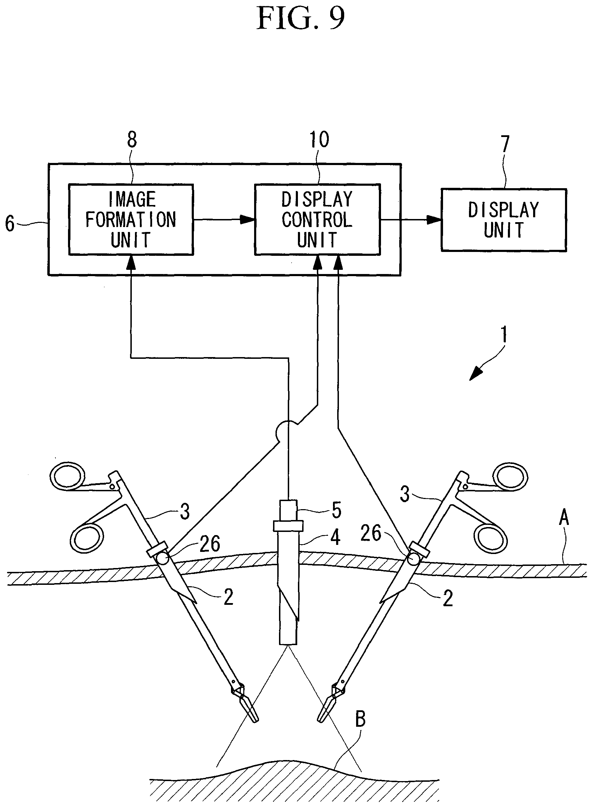

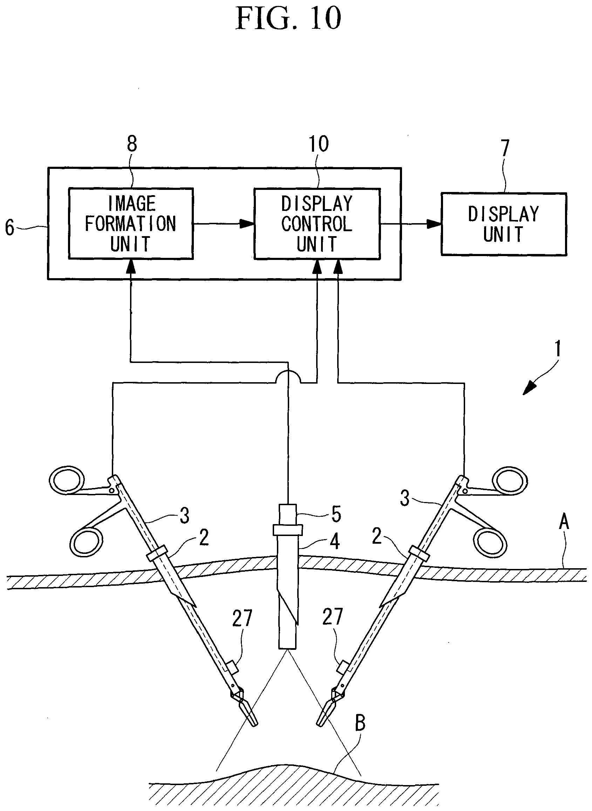

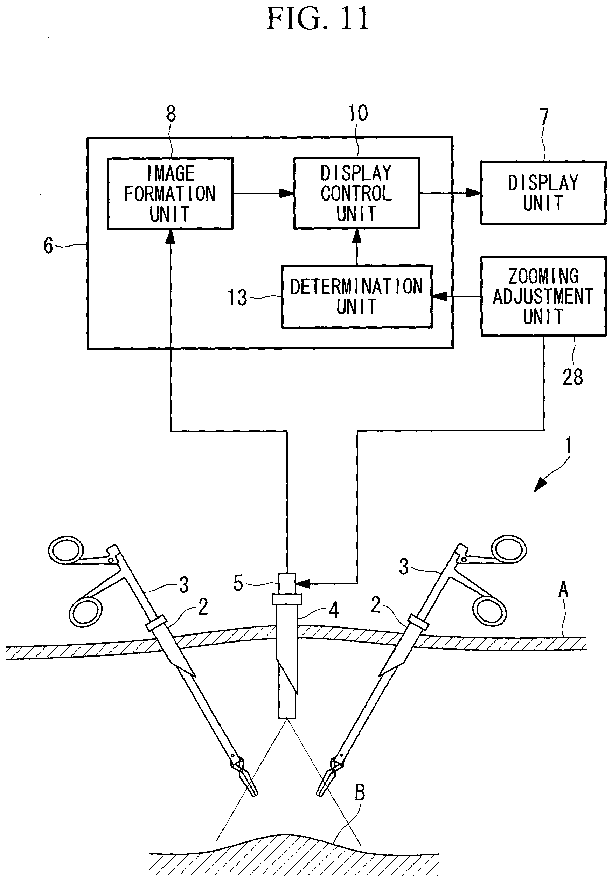

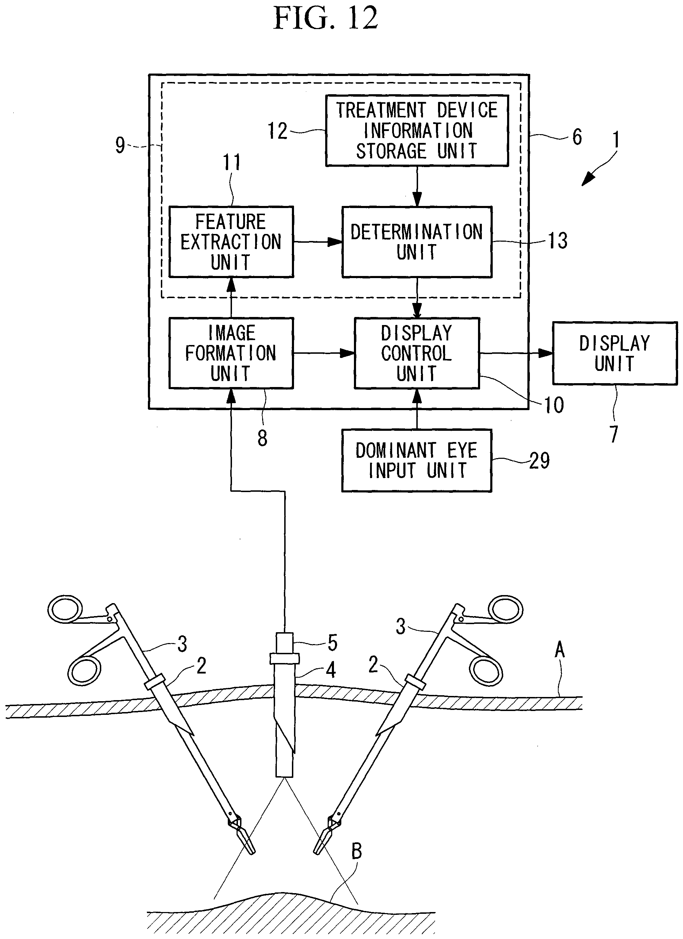

{Fig. 1} Fig. 1 is a schematic diagram of overall configuration showing an image system for surgery according to one embodiment of the present invention.
{Fig. 2} Fig. 2 shows a flowchart for explaining a method for image display according to one embodiment of the present invention using the image system for surgery of Fig. 1.
{Fig. 3} Fig. 3 is a partial enlarged view showing an identification marker provided at a distal end of a treatment device shown as a modification of the image system for surgery of Fig. 1.
{Fig. 4} Fig. 4 shows a flowchart showing a modification of a method for image display by the image system for surgery of Fig. 1.
{Fig. 5} Fig. 5 is a partial enlarged view showing an operation unit of a treatment device showing a contact sensor, which is a modification of the operating state detection unit of the image system for surgery of Fig. 1.
{Fig. 6} Fig. 6 is a diagram showing a modification of the image system for surgery having a contact sensor of Fig. 5.
{Fig. 7} Fig. 7 is a schematic diagram of the overall configuration of the image system for surgery, which is a modification of the image system for surgery of Fig. 1 and comprises a master and slave devices.
{Fig. 8} Fig. 8 is a schematic diagram of overall configuration, which shows an image system for surgery that measures a distance between the treatment device and the operation target portion and which is another modification of the image system for surgery of Fig. 1.
{Fig. 9} Fig. 9 is a schematic diagram of overall configuration of an image system for surgery that detects insertion of the treatment device to a trocar, which is another modification of the image system for surgery of Fig. 1.
{Fig. 10} Fig. 10 is a schematic diagram of overall configuration of the image system for surgery that detects the acceleration of the treatment device, which is another modification of the image system for surgery of Fig. 1.
{Fig. 11} Fig. 11 is a schematic diagram of overall configuration of an image system for surgery that detects magnification of zooming of a three-dimensional imaging unit, which is another modification of the image system for surgery of Fig. 1.
{Fig. 12} Fig. 12 is a schematic diagram of overall configuration of an image system for surgery that displays a two-dimensional image on the side of the dominant eye as a two-dimensional image, which is another modification of the image system for surgery of Fig. 1.

### {Description of Embodiments}

An image system for surgery 1 according to one embodiment of the present invention will be hereafter described with reference to the drawings.

The image system for surgery 1 according to the present embodiment, as shown in Fig. 1, is a system used with a treatment device 3 inserted via a trocar 2 from outside of a body to the inside of the body, and including a three-dimensional imaging unit 5 inserted from outside of a body to the inside of the body via a trocar 4, an image processing unit 6 that outputs and displays an image by processing an image signal acquired by the three-dimensional imaging unit 5, and a display unit 7 that displays an image output by the image processing unit 6. In Fig. 1, reference sign A denotes a body surface tissue, and reference sign B denotes an operation target portion positioned inside of the body.

The three-dimensional imaging unit 5 is a bar-shaped scope in which a camera is disposed that can acquire two images having parallaxes (not shown in the drawings) on the distal end and is insertable to the trocar 4.

The image processing unit 6 comprises an image formation unit 8 that generates an image from an image signal acquired by the three-dimensional imaging unit 5, an operating state detection unit 9 that detects an operating state of the treatment device 3 by processing an image formed by the image formation unit 8, a display control unit 10 that switches the image output to the display unit 7 in accordance with a result of detection by the operating state detection unit 9.

The operating state detection unit 9 comprises a feature extraction unit 11 that extracts a feature within the image sent from the image formation unit 8, a treatment device information storage unit 12 that stores a feature of the treatment device 3, a determination unit 13 that compares the feature extracted by the feature extraction unit 11 and the feature of the treatment device 3 stored in the treatment device information storage unit 12.

The feature extraction unit 11 is configured to, for example, extract features in shape of the treatment device 3 within the image by processing the image. Also, the treatment device information storage unit 12 stores various features in shape of the treatment device 3. The determination unit 13 compares the extracted features in shape and the stored features in shape, and determines whether they agree with each other, in other words, whether the treatment device 3 exists within the image.

The display unit 7 is, for example, a head-mount type display device mounted on the head part of the operator and has two displays (not shown in the drawings) disposed to be opposed to left and right eyes, respectively.

Where it is determined that the treatment device 3 exists within the image by the determination unit 13, the display control unit 10 outputs two images having parallax and formed by the image formation unit 8 so that they are displayed on the right and left displays of the display unit 7, respectively. The display unit 7 can display a stereoscopic image to the operator by separately displaying two images having parallax respectively for left and right images.

On the other hand, where it is determined that the treatment device 3 does not exist within the image by the determination unit 13, the display control unit 10 outputs either one of the two images having parallax and formed by the image formation unit 8 to the right and left displays of the display unit 7 so that they are displayed simultaneously or alternately. The display unit 7 is configured to display two-dimensional image to the operator by displaying the same image on the left and right.

The method for image display by the image system for surgery 1 according to the present embodiment configured as the above will be hereafter described.

In order to perform surgery by using the image system for surgery 1 according to the present embodiment, as shown in Fig. 1, a three-dimensional imaging unit 5 is inserted from the outside of a body to the inside of the body via a trocar 4 installed to penetrate the body surface tissue A of the patient, and dispose the distal end of the three-dimensional imaging unit 5 inserted inside of the body, so as to be opposed to the operation target portion B inside the body.

As shown in Fig. 2, by setting the device to output a two-dimensional image as the output image from the display control unit 10 (step S1), commence capturing an image of the operation target portion B by the three-dimensional imaging unit 5 (step S2). When an image signal is acquired by the three-dimensional imaging unit 5, the acquired image signal having the parallax is sent to an image formation unit 8 of the image processing image processing unit 6, where two images having parallax are formed. Since the display control unit 10 is set to output a two-dimensional image, the right and left displays of the display unit 7 displayed either one of the acquired two images having parallax simultaneously or alternately (step S3). This allows the operator to observe the operation target portion B with a two-dimensional image.

When images with parallax are formed by the image formation unit 8, one of the images is sent to the operating state detection unit 9. In the operating state detection unit 9, the sent image is processed in the feature extraction unit 11. Thereby, features within the image are extracted (step S4), and the extracted features are sent to the determination unit 13. In the determination unit 13, the features sent from the feature extraction unit 11 and various features of the treatment device 3 stored in the treatment device information storage unit 12 are compared to determine whether the features extracted from within the image indicate the treatment device 3 (step S5).

As a result of determination, where it is determined that the treatment device 3 exists within the image, the display control unit 10 controls the display unit 7 so as to display two images having parallax and sent from the image formation unit 8 on the two displays of the display unit 7 opposed to both left and right eyes (step S7). As a result, the operator can observe the operation target portion B with a stereoscopic image.

Appearance of the treatment device 3 within the image is the representation of the intention of the operator to operate the treatment device 3 to initiate the treatment operation of the target portion B. By switching the image displayed on the display unit 7 to the stereoscopic image at this time point, it is possible to provide the operator with the stereoscopic image by which the operator can easily obtain sense of depth on performing the treatment.

Contrarily, where there is no treatment device 3 within the image, the operator is not about to initiate treatment by operating the treatment device 3, and it is possible to provide a two-dimensional image providing less sense of depth to the operator (step S6). Where it is determined whether the observation is completed, and it is not the observation completion, the processes from step S2 are repeated (step S8).

In this way, according to the image system for surgery 1 according to the present embodiment, it becomes possible to allow the operator to observe the operation target portion B with a two-dimensional image in a state where the treatment device 3 has not appeared within the image, and observe the operation target portion B with the stereoscopic image when the treatment device 3 has appeared within the image. This configuration provides a benefit of preventing the operator from continuously observing the stereoscopic image over and extended period even when the surgery continued over an extended period, and, reducing the fatigue of the operator.

In the present embodiment, the features in shape of the treatment device 3 are extracted with the feature extraction unit 11, they are compared in the determination unit 13 with a variety of features in shape of the treatment device 3 stored in the treatment device information storage unit 12, presence or absence of the treatment device 3 within the image is determined. Instead of this configuration, however, it may be possible to configure the structure so that, as shown in Fig. 3, an identification marker 14, such as a bar code or a two dimension bar code may be adhered near the distal end of the treatment device 3 and the feature extraction unit 11 may extract the identification marker 14 and store the identification marker 14 and identification information in association with one another in the treatment device information storage unit 12.

With this configuration, it is possible to more easily determine the presence of the treatment device 3 within the image than the case where comparison is performed by comparing the features in shape of the treatment device 3 by recognizing them.

Further, by adding associated information to the identification marker 14, such as a bar code, it is possible to store, in addition to the identification information of the treatment device 3, another associated information, such as use state of the treatment device 3, etc. in association with one another.

Further, as the identification marker 14 a color not present inside of the body may be used instead of the bar code.

Further, in the present embodiment, as an operating state detection unit 9 that detects the operating state in which the treatment device 3 initiates the treatment of the operation target portion B, it is detected whether the treatment device 3 exists within the image. However, instead of this configuration, it may be detected whether the treatment device 3 extracted within the image has moved.

In this case, as shown in Fig. 4, when the treatment device 3 is detected in step S5, the position of the treatment device 3 within the image is stored (step S10), and where the position of the treatment device 3 is already stored, the amount of movement to the position newly stored is calculated (step S11). It is determined whether the calculated amount of movement exceeds a predetermined threshold (step S12), the system switches to displaying a stereoscopic image where the amount of movement is more than the threshold (step S7), and switches to displaying a two-dimensional image where the amount of movement is equal to or less than the threshold (step S6).

In the image, where the treatment device 3 moves, it is more apparent that the treatment device 3 is in the operating state in which the operator initiates the treatment of the operation target portion B. By detecting such a state and switching to displaying a stereoscopic image, it is possible to make it easier to obtain the sense of depth on performing the treatment and make the treatment easy.

Further, in the present embodiment, by processing the acquired image, displaying is switched between a stereoscopic image and a two-dimensional image, depending on the presence or displacement of the treatment device 3. Instead of this, however, as shown in Fig. 5, by providing a contact sensor 15 to the operation unit 3a for operating the treatment device 3, and detecting that the operator has grasped the operation unit 3a with the contact sensor 15, the system may detect the treatment device 3 turned to the operating state in which to initiate the treatment of the operation target portion B. In this case, as shown in Fig. 6, the outputs from the contact sensor 15 may be input directly to the display control unit 10, and where the contact on the operation unit 3a is detected by the contact sensor 15, the system may switch to displaying a stereoscopic image.

With this configuration, it is possible to eliminate necessity of the image processing or recognition of the shape of the treatment device 3, and achieve easier and more direct detection of being in the operating state of the treatment device 3 in which to initiate the treatment of the operation target portion B.

A pressure sensor may be adopted instated of the contact sensor. In this case, the system may switch between displaying a stereoscopic image and displaying a two-dimensional image depending on a value of pressure applied to a pressure sensor.

As an operation unit 3a of the treatment device 3, the treatment device 3 and an operation unit of the treatment device (not shown in the drawings) may be installed on a system of master-slave scheme as shown in Fig. 7, in addition to the operation unit 3a as shown in Fig. 6 connected to the treatment device 3 for directly operating the treatment device 3. In this case, the treatment device 3 is installed on the manipulator on the slave side (slave manipulator) 16, the operation unit is installed on the manipulator (master manipulator) 17 on the master side. In this configuration, a contact sensor 15 may be provided to the operation unit.

Also in this case, where the operator operates the operation part on the master manipulator 17, the slave manipulator 16 operates to set the treatment device 3 in the operating state to initiate the treatment. Therefore, the system may switch image displaying by detecting operation to the operation unit of the master manipulator 17 by the contact sensor 15.

In the example shown in Fig. 7, on the slave side on which an operating table 18 on which patient C is placed is disposed, two treatment devices 3 are respectively mounted on two slave manipulators 16. The three-dimensional imaging unit 5 is mounted on an observation manipulator 19 on the slave side.

On the master side, a master manipulator 17 to which the operator performs operation, a display unit 7, such as a head mount display for providing the operator with an image, a digitizer 20 that detects the direction of view field of the operator are disposed, and a contact sensor 15 for detecting the operation to the operation unit by the operator is provided on the master manipulator 17.

Between the master side and the slave side is displaced a control memory 21. The control memory 21 comprises a treatment device control unit 22 that controls a slave manipulator 16 and the treatment device 3 based on a signal from the master manipulator 17, an observation part control unit 23 that controls the observation manipulator 19 based on a signal from the digitizer 20, and an image control unit 6 comprising an image formation unit 8 that generates two images having parallax by processing an image signal acquired by the three-dimensional imaging unit 5, and a display control unit 10 that switches images between the stereoscopic image and the two-dimensional image according to the signal from the contact sensor 15 and outputs the switched image to the display unit 7.

Although the contact sensor 15 is exemplified as a sensor for detecting operation to the operation unit of the master manipulator 17 by the operator, a sensor described below may be adopted in place of this.

A pressure sensor: detects a pressure by which the operation unit is gripped and the system switches to a stereoscopic image where the pressure is greater than the threshold, and switches to a two-dimensional image where the pressure is less than the threshold.

A holding finger sensor: a contact sensor 15 is provided for each holding finger, and the system switches to displaying a stereoscopic image where three fingers are in contact with the sensor, and a two-dimensional image where two fingers are in contact with the sensor.

A contact sensor on the arm rest of the master manipulator 17 (not shown in the drawings): The system switches to displaying a stereoscopic image where the operator put their elbow on the arm rest in order to operate the master manipulator 17, and switches to displaying a two-dimensional image where their elbow is not put on the arm rest.

A sensor for detecting a specific operation pattern on the operation unit: For example, the system detects the operation pattern in which the operation unit is operated two times in succession, and switches between the stereoscopic image and the two-dimensional image each time the pattern is detected. Or, an operation pattern may be detected that is not performed on a normal operation on the treatment.

A sensor for detecting a stroke of the master manipulator 17: The system switches to displaying the stereoscopic image where the stroke exceeds the threshold, and switches to displaying the two-dimensional image where the stroke is threshold or less. The image may be switched to the stereoscopic image where the stroke direction is such a direction to put the treatment device 3 closer to the operation target portion B.

Further, a contact sensor (not shown in the drawings) may be provided on the two treatment devices 3, and the image may be switched each time the contact is detected.

Further, in Fig. 1, the determination unit 13 determines presence or absence of the treatment device 3 within the image. However, instead of this, after extraction of the treatment device 3 within the image, the positional relationship of the treatment device 3 and the operation target portion B in the depth direction within the image may be calculated (stereophonic measuring) based on the two images having parallax, and it may be determined whether the distance between the distal end of the treatment device 3 and the operation target portion B exceeds the threshold. As a result of determination, the system may switch to displaying a stereoscopic image where they have come close to each other by exceeding the threshold, or may switch to a two-dimensional image where the distance is the threshold or greater.

The method of stereophonic measuring may use, for example, a method that acquires three-dimensional information by publicly known polygons or wire frames as disclosed in Japanese Unexamined Patent Application Publication No. 2003-6616. Further, an easily identifiable marker may be provided on the distal end of the treatment device 3. By configuring as above, it is possible to calculate the positional relationship in the depth direction between the marker and the operation target portion B without recognizing the shape of the treatment device 3.

In the above, the position of the operation target portion B is detected based on the two images having parallax and acquired by the three-dimensional imaging unit 5. However, instead of this, the system may calculate the distance between the treatment device 3 and the operation target portion B by utilizing the positional information of the operation target portion B measured in advance by an MRI apparatus 24 or the like, as shown in Fig. 8. In the example shown in Fig. 8, a gyro sensor 25 is disposed in the treatment device 3 and the three-dimensional imaging unit 5, and the output of the gyro sensor 25 and the output from the MRI apparatus 24 are input to the determination unit 13. The determination unit 13, based on the position of the treatment device 3 detected by the gyro sensor 25 and the position of the operation target portion B input from the MRI apparatus 24, calculates the distance therebetween and compares it with the threshold to switch displaying the image.

Further, as shown in Fig. 9, the sensor 26 for detecting the passage of the treatment device 3 at the trocar 2 for inserting the treatment device 3 inside of the body, and where the passage of the treatment device 3 is detected by the sensor 26, the display control unit 10, may switch the image to be displayed on the display unit 7 from a two-dimensional image to a stereoscopic image.

Further, as shown in Fig. 10, an acceleration sensor 27 may be provided on the distal end of the treatment device 3, and where acceleration of a predetermined magnitude or more is detected, determines that the treatment device 3 is in the operating state in which to initiate the treatment of the operation target portion B, and the display control unit 10 may switch the image to be displayed on the display unit 7 from a two-dimensional image to a stereoscopic image. The amount of displacement may be calculated based on the acceleration detected by the acceleration sensor 27, and where the amount of displacement has become a predetermined value or greater, it may be determined that the system is in the operating state, and displaying may be switched from a two-dimensional image to a stereoscopic image.

In each of the above-described embodiments, it is determined by various methods whether the treatment device 3 is in the operating state in which to initiate the treatment on the operation target portion B and displaying an image is switched. However, instead of this, it may be possible to determine whether the three-dimensional imaging unit 5 is in the operating state in which to initiate treatment on the operation target portion B.

For example, as shown in Fig. 11, the determination unit 13 may determine whether the operator has set magnification of zooming to be greater than a predetermined threshold by operating the zooming adjustment unit 28 where the set magnification of zooming exceeds the predetermined threshold, the display control unit 10 may switch displaying from a two-dimensional image to displaying a stereoscopic image, and may switch from displaying a stereoscopic image to a two-dimensional image where the magnification is the threshold or less.

Where the magnification of zooming is set large by the operator, it is possible to regard the situation as one in which the operator is about to perform the treatment while precisely observing the operation target portion B by magnifying the operation target portion B. Accordingly, by switching displaying of an image on the display unit 7 to a stereoscopic image only in this case, it is possible to increase the easiness of treatment while minimizing the fatigue accompanied by observing the stereoscopic image over the extended period.

The display control unit 10, in addition to switching displaying from a two-dimensional image to a stereoscopic image where it is determined that the magnification of zooming is larger than the predetermined threshold by the determination unit 13, may maintain the image to be displayed on the display unit 7 as a two-dimensional image during the operation of switching the magnification of zooming. With this configuration, it is possible to prevent the operator from feeling dizziness by observing the stereoscopic image during change of the magnification of zooming.

Further, while the display is switched depending on the magnification of zooming by the operation of the zooming adjustment unit 28 by the operator, it may be possible, instead of this, to determine the state of the operation target portion B, such as, for example, the size of a tumor, etc. by image processing, and switch to displaying a stereoscopic image where the magnification of zooming is changed automatically to an appropriate value. Here, an appropriate magnification of zooming depending on the treatment device 3 to be used may be selected as the appropriate magnification of zooming.

Further, in the above description, a two-dimensional image and a stereoscopic image are switched from one another by various methods. However, as shown in Fig. 12, there may be provided a dominant eye input unit 29 through which the operator inputs their dominant eye. It is preferable to switch to displaying a two-dimensional image on the side of the dominant eye on the display unit 7 when, by sending the input information of the dominant eye to the display control unit 10, the display control unit 10 switches to displaying a two-dimensional image.

With this configuration, there is an advantage that the operator can observe the image without any uncomfortable feeling since no offset is caused in the side of the dominant eye of the operator in the image when the display is switched to the two-dimensional image during the observation by the stereoscopic image. The same advantage may be obtained also when the display is switched from a two-dimensional image to a stereoscopic image, which is the case inverse to the above. A storage unit that stores the information on the dominant eye may serve as the dominant eye input unit 29.

In the above description, one of the images selected from the two images acquired by the three-dimensional imaging unit is presented to both eyes when the two-dimensional image is displayed. However, any element may be employed as long as it presents to both eyes a same image, for example, one obtained by performing 2D image synthesis on two images.

### {Reference Signs List}

B operation target portion
1 image system for surgery
2 trocar
3a operation unit
5 three-dimensional imaging unit
7 display unit
9 operating state detection unit
10 display control unit
13 determination unit (operating state detection unit)
15 contact sensor (operating state detection unit)
17 master manipulator (master device)
24 MRI apparatus (operating state detection unit)
25 gyro sensor (operating state detection unit)
26 sensor (operating state detection unit)
27 acceleration sensor (operating state detection unit)
28 zooming adjustment element (operating state detection unit)
29 dominant eye input unit (storage unit)

## Claims

1. An image system (1) for surgery, the image system (1) comprising:
a three-dimensional imaging unit (5) configured to acquire two images having parallax by capturing an image of an operation target portion;
a display unit (7) configured to display the two images acquired by the three-dimensional imaging unit (5) individually for both left and right eyes, or display a same image for both left and right eyes;
an operating state detection unit (9) configured to detect that a treatment device (3) that performs treatment on the operation target portion or the three-dimensional imaging unit (5) is in an operating state in which to initiate treatment on the operation target portion; and **characterized by**
a display control unit (10) configured to switch from displaying the same one image on the display unit (7) to displaying the two images having parallax on the display unit (7) when the operating state detection unit (9) detects that the three-dimensional imaging unit (5) or the treatment device (3) is in the operating state in which to initiate treatment on the operation target portion.

2. The image system (1) for surgery according to claim 1, wherein the operating state detection unit (9) is configured to detect displacement, velocity or acceleration of the treatment device (3).

3. The image system (1) for surgery according to claim 1 or 2, wherein the operating state detection unit (9) is configured to determine that the treatment device (3) is in the operating state in which to initiate the treatment on the operation target portion when the operating state detection unit (9) processes any of the images acquired by the three-dimensional imaging unit (5), and the treatment device (3) is detected within the image.

4. The image system (1) for surgery according to claim 2, wherein the operating state detection unit (9) is configured to process any of the images acquired by the three-dimensional imaging unit (5) and calculate displacement, velocity or acceleration of the treatment device (3) within the image, and determine that the treatment device (3) is in the operating state in which to initiate the treatment on the operation target portion when the calculated displacement, velocity or acceleration exceeds a predetermined threshold.

5. The image system (1) for surgery according to claim 1, wherein the operating state detection unit (9) is provided on an operation unit (3a) for operating the treatment device (3), and is configured to detect contact on the operation unit (3a) or the operation of the operation unit (3a).

6. The image system (1) for surgery according to claim 5, wherein the operation unit (3a) is provided on a master device disposed at a position remote from the treatment device (3) and is configured to operate the treatment device (3) by remote operation.

7. The image system (1) for surgery according to claim 5 or 6, wherein the operating state detection unit (9) is configured to detect an amount of displacement or a direction of displacement of the operation unit (3a), and in a case where the detected amount of displacement exceeds a predetermined threshold or where the detected direction of displacement is a predetermined direction of displacement, determine that the treatment device (3) is in an operating state in which to initiate treatment on the operation target portion.

8. The image system (1) for surgery according to claim 1, wherein the operating state detection unit is provided on a trocar for allowing insertion of the treatment device (3) from outside of a body to inside of the body, and when a distal end of the treatment device (3) is inserted inside of the body via the trocar, is configured to determine that the treatment device (3) is in the operating state in which to initiate treatment on the operation target portion.

9. The image system (1) for surgery according to claim 1, wherein the operating state detection unit (9) is configured to measure a distance between a distal end position of the treatment device (3) and the operation target portion, and when the measured distance is smaller than a predetermined threshold, is configured to determine that the treatment device (3) is in the operating state in which to initiate treatment on the operation target portion.

10. The image system (1) for surgery according to claim 1, wherein
the three-dimensional imaging unit (5) includes a zooming optical system,
the operating state detection unit (9) is configured to detect an magnification of the zooming optical system, and determine that the treatment device (3) is in an operating state in which to initiate treatment on the operation target portion when it is detected that the magnification exceeds a predetermined threshold.

11. The image system (1) for surgery according to any one of claims 1 to 10, further comprising
a storage unit (12) configured to store dominant eye information on which eye of an operator the dominant eye is,
wherein the display control unit (10) is configured to, when it is not detected that the three-dimensional imaging unit (5) or the treatment device (3) is in the operating state in which to initiate treatment on the operation target portion, display the image on the dominant eye side on the display unit (7) based on dominant eye information stored in the storage unit (12).

## Patentansprüche

1. Bildsystem (1) für die Chirurgie, wobei das Bildsystem (1) umfasst:
eine dreidimensionale Bildgebungseinheit (5), die so konfiguriert ist, dass sie zwei Bilder mit Parallaxe durch Aufnehmen eines Bilds eines Operationszielabschnitts erfasst;
eine Anzeigeeinheit (7), die so konfiguriert ist, dass sie die zwei von der dreidimensionalen Bildgebungseinheit (5) erfassten Bilder sowohl für das linke als auch das rechte Auge individuell anzeigt oder ein gleiches Bild für das linke und das rechte Auge anzeigt;
eine Betriebsstatuserkennungseinheit (9), die so konfiguriert ist, dass sie erkennt, dass eine Behandlungsvorrichtung (3), die eine Behandlung am Operationszielabschnitt durchführt, oder die dreidimensionale Bildgebungseinheit (5) sich in einem Betriebsstatus befindet, in dem eine Behandlung am Operationszielabschnitt einzuleiten ist; und **gekennzeichnet ist durch**:
eine Anzeigesteuereinheit (10), die so konfiguriert ist, dass sie von einem Anzeigen des gleichen einen Bilds auf der Anzeigeeinheit (7) zu einem Anzeigen der zwei Bilder mit Parallaxe auf der Anzeigeeinheit (7) wechselt, wenn die Betriebsstatuserkennungseinheit (9) erkennt, dass die dreidimensionale Bildgebungseinheit (5) oder die Behandlungsvorrichtung (3) sich in dem Betriebsstatus befindet, in dem eine Behandlung am Operationszielabschnitt einzuleiten ist.

2. Bildsystem (1) für die Chirurgie nach Anspruch 1, wobei die Betriebsstatuserkennungseinheit (9) so konfiguriert ist, dass sie eine Verschiebung, eine Geschwindigkeit oder eine Beschleunigung der Behandlungsvorrichtung (3) erkennt.

3. Bildsystem (1) für die Chirurgie nach Anspruch 1 oder 2, wobei die Betriebsstatuserkennungseinheit (9) so konfiguriert ist, dass sie ermittelt, dass die Behandlungsvorrichtung (3) sich in dem Betriebsstatus befindet, in dem die Behandlung am Operationszielabschnitt einzuleiten ist, wenn die Betriebsstatuserkennungseinheit (9) beliebige der von der dreidimensionalen Bildgebungseinheit (5) erfassten Bilder verarbeitet und die Behandlungsvorrichtung (3) innerhalb des Bilds erkannt wird.

4. Bildsystem (1) für die Chirurgie nach Anspruch 2, wobei die Betriebsstatuserkennungseinheit (9) so konfiguriert ist, dass sie beliebige der von der dreidimensionalen Bildgebungseinheit (5) erfassten Bilder verarbeitet und eine Verschiebung, eine Geschwindigkeit oder eine Beschleunigung der Behandlungsvorrichtung (3) innerhalb des Bilds berechnet und ermittelt, dass die Behandlungsvorrichtung (3) sich in dem Betriebsstatus befindet, in dem die Behandlung am Operationszielabschnitt einzuleiten ist, wenn die berechnete Verschiebung, Geschwindigkeit oder Beschleunigung einen vordefinierten Schwellenwert überschreitet.

5. Bildsystem (1) für die Chirurgie nach Anspruch 1, wobei die Betriebsstatuserkennungseinheit (9) auf einer Betriebseinheit (3a) zum Betreiben der Behandlungsvorrichtung (3) vorgesehen und so konfiguriert ist, dass sie einen Kontakt auf der Betriebseinheit (3a) oder den Betrieb der Betriebseinheit (3a) erkennt.

6. Bildsystem (1) für die Chirurgie nach Anspruch 5, wobei die Betriebseinheit (3a) auf einer übergeordneten Vorrichtung vorgesehen ist, die in einer von der Behandlungsvorrichtung (3) entfernt gelegenen Position angeordnet und so konfiguriert ist, dass sie die Behandlungsvorrichtung (3) durch Fernbetrieb betreibt.

7. Bildsystem (1) für die Chirurgie nach Anspruch 5 oder 6, wobei die Betriebsstatuserkennungseinheit (9) so konfiguriert ist, dass sie einen Verschiebungsbetrag oder eine Verschiebungsrichtung der Betriebseinheit (3a) erkennt, und dass sie, wenn der erkannte Verschiebungsbetrag einen vordefinierten Schwellenwert überschreitet oder die erkannte Verschiebungsrichtung eine vordefinierte Verschiebungsrichtung ist, ermittelt, dass die Behandlungsvorrichtung (3) sich in einem Betriebsstatus befindet, in dem eine Behandlung am Operationszielabschnitt einzuleiten ist.

8. Bildsystem (1) für die Chirurgie nach Anspruch 1, wobei die Betriebsstatuserkennungseinheit auf einem Trokar vorgesehen ist, um das Einführen der Behandlungsvorrichtung (3) von außerhalb eines Körpers in das Innere des Körpers zu ermöglichen, und so konfiguriert ist, dass sie, wenn ein distales Ende der Behandlungsvorrichtung (3) über den Trokar in das Innere des Körpers eingeführt ist, ermittelt, dass die Betriebsvorrichtung (3) sich in dem Betriebszustand befindet, in dem eine Behandlung am Operationszielabschnitt einzuleiten ist.

9. Bildsystem (1) für die Chirurgie nach Anspruch 1, wobei die Betriebsstatuserkennungseinheit (9) so konfiguriert ist, dass sie einen Abstand zwischen einer distalen Endposition der Behandlungsvorrichtung (3) und dem Operationszielabschnitt misst, und so konfiguriert ist, dass sie, wenn der gemessene Abstand kleiner als ein vordefinierter Schwellenwert ist, ermittelt, dass die Behandlungsvorrichtung (3) sich in dem Betriebsstatus befindet, in dem eine Behandlung am Operationszielabschnitt einzuleiten ist.

10. Bildsystem (1) für die Chirurgie nach Anspruch 1, wobei:
die dreidimensionale Bildgebungseinheit (5) ein optisches Zoomsystem umfasst,
die Betriebsstatuserkennungseinheit (9) so konfiguriert ist, dass sie eine Vergrößerung des optischen Zoomsystems erkennt und ermittelt, dass die Behandlungsvorrichtung (3) sich in einem Betriebsstatus befindet, in dem eine Behandlung am Operationszielabschnitt einzuleiten ist, wenn erkannt wird, dass die Vergrößerung einen vordefinierten Schwellenwert überschreitet.

11. Bildsystem (1) für die Chirurgie nach einem der Ansprüche 1 bis 10, das ferner umfasst:
eine Speichereinheit (12), die so konfiguriert ist, dass sie eine Information zum dominanten Auge in Bezug darauf, welches Auge eines Benutzers das dominante Auge ist, speichert,
wobei die Anzeigesteuereinheit (10) so konfiguriert ist, dass sie, wenn nicht erkannt wird, dass die dreidimensionale Bildgebungseinheit (5) oder die Behandlungsvorrichtung (3) sich im Betriebsstatus befinden, in dem eine Behandlung am Operationszielabschnitt durchzuführen ist, das Bild auf Basis der in der Speichereinheit (12) gespeicherten Information zum dominanten Auge auf der Seite des dominanten Auges auf der Anzeigeeinheit (7) anzeigt.

## Revendications

1. Système d'imagerie (1) pour une opération chirurgicale, le système d'imagerie (1) comprenant :
une unité d'imagerie tridimensionnelle (5) configurée pour acquérir deux images ayant une parallaxe par capture d'une image d'une partie de cible d'opération ;
une unité d'affichage (7) configurée pour afficher les deux images acquises par l'unité d'imagerie tridimensionnelle (5) individuellement pour à la fois les yeux gauche et droit, ou afficher une même image pour à la fois les yeux gauche et droit ;
une unité de détection d'état de fonctionnement (9) configurée pour détecter qu'un dispositif de traitement (3) qui réalise un traitement sur la partie de cible d'opération ou l'unité d'imagerie tridimensionnelle (5) est dans un état de fonctionnement dans lequel un traitement est initié sur la partie de cible d'opération ; et **caractérisé par**
une unité de commande d'affichage (10) configurée pour commuter de l'affichage de la même image sur l'unité d'affichage (7) à l'affichage des deux images ayant une parallaxe sur l'unité d'affichage (7) lorsque l'unité de détection d'état de fonctionnement (9) détecte que l'unité d'imagerie tridimensionnelle (5) ou le dispositif de traitement (3) est dans l'état de fonctionnement dans lequel un traitement est initié sur la partie de cible d'opération.

2. Système d'imagerie (1) pour une opération chirurgicale selon la revendication 1, dans lequel l'unité de détection d'état de fonctionnement (9) est configurée pour détecter un déplacement, une vitesse ou une accélération du dispositif de traitement (3).

3. Système d'imagerie (1) pour une opération chirurgicale selon la revendication 1 ou 2, dans lequel l'unité de détection d'état de fonctionnement (9) est configurée pour déterminer que le dispositif de traitement (3) est dans l'état de fonctionnement dans lequel un traitement est initié sur la partie de cible d'opération lorsque l'unité de détection d'état de fonctionnement (9) traite l'une quelconque des images acquises par l'unité d'imagerie tridimensionnelle (5), et le dispositif de traitement (3) est détecté dans l'image.

4. Système d'imagerie (1) pour une opération chirurgicale selon la revendication 2, dans lequel l'unité de détection d'état de fonctionnement (9) est configurée pour traiter l'une quelconque des images acquises par l'unité d'imagerie tridimensionnelle (5) et calculer un déplacement, une vitesse ou une accélération du dispositif de traitement (3) dans l'image, et déterminer que le dispositif de traitement (3) est dans l'état de fonctionnement dans lequel un traitement est initié sur la partie de cible d'opération lorsque le déplacement, la vitesse ou l'accélération calculé(e) excède un seuil prédéterminé.

5. Système d'imagerie (1) pour une opération chirurgicale selon la revendication 1, dans lequel l'unité de détection d'état de fonctionnement (9) est disposée sur une unité de commande (3a) pour commander le dispositif de traitement (3), et est configurée pour détecter un contact sur l'unité de commande (3a) ou le fonctionnement de l'unité de commande (3a).

6. Système d'imagerie (1) pour une opération chirurgicale selon la revendication 5, dans lequel l'unité de commande (3a) est disposée sur un dispositif maître disposé au niveau d'un emplacement éloigné du dispositif de traitement (3) et est configurée pour commander le dispositif de traitement (3) par un fonctionnement à distance.

7. Système d'imagerie (1) pour une opération chirurgicale selon la revendication 5 ou 6, dans lequel l'unité de détection d'état de fonctionnement (9) est configurée pour détecter une quantité de déplacement ou une direction de déplacement de l'unité de commande (3a), et dans un cas dans lequel la quantité détectée de déplacement dépasse un seuil prédéterminé ou dans lequel la direction détectée de déplacement est une direction prédéterminée de déplacement, déterminer que le dispositif de traitement (3) est dans un état de fonctionnement dans lequel un traitement est initié sur la partie de cible d'opération.

8. Système d'imagerie (1) pour une opération chirurgicale selon la revendication 1, dans lequel l'unité de détection d'état de fonctionnement est disposée sur un trocart pour permettre une insertion du dispositif de traitement (3) depuis l'extérieur d'un corps vers l'intérieur du corps, et lorsqu'une extrémité distale du dispositif de traitement (3) est insérée à l'intérieur du corps par l'intermédiaire du trocart, est configurée pour déterminer que le dispositif de traitement (3) est dans l'état de fonctionnement dans lequel un traitement est initié sur la partie de cible d'opération.

9. Système d'imagerie (1) pour une opération chirurgicale selon la revendication 1, dans lequel l'unité de détection d'état de fonctionnement (9) est configurée pour mesurer une distance entre un emplacement d'extrémité distale du dispositif de traitement (3) et la partie de cible d'opération, et lorsque la distance mesurée est inférieure à un seuil prédéterminé, est configurée pour déterminer que le dispositif de traitement (3) est dans l'état de fonctionnement dans lequel un traitement est initié sur la partie de cible d'opération.

10. Système d'imagerie (1) pour une opération chirurgicale selon la revendication 1, dans lequel
l'unité d'imagerie tridimensionnelle (5) comprend un système optique de variation de focale,
l'unité de détection d'état de fonctionnement (9) est configurée pour détecter un grossissement du système optique de variation de focale, et déterminer que le dispositif de traitement (3) est dans un état de fonctionnement dans lequel un traitement est initié sur la partie de cible d'opération lorsqu'il est détecté que le grossissement dépasse un seuil prédéterminé.

11. Système d'imagerie (1) pour une opération chirurgicale selon l'une quelconque des revendications 1 à 10, comprenant en outre
une unité de stockage (12) configurée pour stocker des informations d'oeil dominant sur lesquelles un oeil d'un opérateur est l'oeil dominant,
dans lequel l'unité de commande d'affichage (10) est configurée pour, lorsqu'il n'est pas détecté que l'unité d'imagerie tridimensionnelle (5) ou le dispositif de traitement (3) est dans l'état de fonctionnement dans lequel un traitement est initié sur la partie de cible d'opération, afficher l'image sur le côté d'oeil dominant sur l'unité d'affichage (7) sur la base d'informations d'oeil dominant stockées dans l'unité de stockage (12).
